# EUROPEAN PATENT APPLICATION

(11) **EP 1 790 283 A1**
(43) Date of publication of application: **30.05.2007**
(21) Application number: 06256051.1
(22) Date of filing: 27.11.2006
(51) Int. Cl.: A61B 5/00, A61B 5/0275

(54) **Method and system of measuring blood circulation velocity**

(30) Priority: 28.11.2005 CN 200510126927
(71) Applicant: Zen-u Biotechnology Co., Ltd, Sindian City, Taipei 231 (TW)
(72) Inventor: Lu, Hsueh-Kuan, Taichung City 400 (TW); Lu, Chih-YI, Chiayi County 606 (TW)
(74) Representative: Prentice, Raymond Roy

(57) **Abstract**

A system and method of measuring blood circulation velocity includes the steps of keeping a person measured by a blood oxygen saturation analytical instrument and served by a ventilator; setting an initial time point, providing a first gas to the person via the ventilator, and starting to record a blood oxygen saturation value per predict time interval; providing a second gas to the person at a first time point; providing a third gas to the person at a second time point; stopping recording the blood oxygen saturation value at a terminal time point; setting a reference time point according to the blood oxygen saturation value which has a variation according to the records; obtaining the person's blood circulation velocity, which is proportional to the reciprocal of the difference of the reference time point and the first time point..

## Description

### BACKGROUND

The present invention relates to methods of measuring blood circulation velocity for obtaining a person's blood circulation velocity, and more particularly to a method of measuring blood circulation velocity by controlling breath of the person to be measured. The present invention also provides a system applying the method.

In recent years, when people's health is getting worse, they start valuing the related subject of heath. People look for treat by food, physics treatment or medicine cure method and etc., trying to improve their healthy condition. However, the health has no absolute quantity index, the people usually regard the quality of their blood circulation condition as one kind of reference index. In other words, when blood circulation condition is not well, the healthy condition may not be well either; vice versa, the healthy condition might be also well.

However, how to exactly judge the blood circulation condition of a person is well or not? One kind of conventional method is by injecting photosensitive matter into the person's blood firstly, then utilizing photosensitive photograph or taking photo to observe blood circulation of the person to be measured. Nevertheless, this way can only acquire blood circulation condition to be measured once, but can not repeat to obtain blood circulation velocity quantity.

In addition, another conventional method is using a blood current velocity measurement manufactured according to the principle of the Dopplor effect. The theory of this method is that the flowing blood may induce the anti-diffusing photons to generate Dopplor frequency shift, and the quantity of the Dopplor frequency shift is proportional to the blood current velocity; so using an Optical Doppler Tomography (ODT) to scan the Dopplor frequency shift would obtain the blood current velocity. However, this method is not easy to carry out, it is because that although each Dopplor frequency shift is certain corresponding to a particular object, the fluxion of blood is continuous, therefore; the measurement value isn't accurate and the emersion is lower. Moreover, the scanning instrument, such as Optical Doppler Tomography, is expensive, so this conventional method isn't widespread.

Accordingly, what is needed is a method of measuring blood circulation velocity that can overcome the above-described deficiencies.

### BRIEF SUMMARY

The present invention provides a method and a system of measuring blood circulation velocity, and the method is realized by controlling a person's breath and using a blood oxygen saturation analytical instrument to rapidly measuring and obtaining blood circulation velocity of the person to be measured.

The method of measuring blood circulation velocity includes the steps of keeping a person measured by a blood oxygen saturation analytical instrument and served by a ventilator; setting an initial time point, providing a first gas to the person via the ventilator, and starting to record a blood oxygen saturation value per predict time interval; providing a second gas to the person at a first time point; providing a third gas to the person at a second time point; stopping recording the blood oxygen saturation value at a terminal time point; setting a reference time point according to the blood oxygen saturation value which has a variation according to the records; obtaining the person's blood circulation velocity, which is proportional to the reciprocal of the difference of the reference time point and the first time point.

According to the above-mentioned method, the fist gas is different to the second gas, in which, the first gas can be selected from the group of oxygen, air, and the combination of oxygen and air; and the second gas can be selected form the group of carbon monoxide (CO), nitrogen (N2), carbon dioxide (CO2), the gas without oxygen, and the combination thereof.

According to the above-mentioned method, the blood circulation velocity of the person to be measured is obtained by using a reference distance dividing the difference of the reference time point and the first time point, wherein the reference distance is proportional to the height of the person to be measured, and then which may be corrected according to the height of the person to be measured.

According to the above-mentioned method, if the blood oxygen saturation value at the first time point is different to that at the initial time point, then make the record of the blood oxygen saturation value invalid.

The system of measuring the blood circulation velocity includes a main controller; a blood oxygen saturation analytical instrument electrically connecting with the main controller for detecting the blood oxygen saturation value of the person to be measured per predict time interval and transmitting the blood oxygen saturation value to the main controller; a ventilator electrically connecting with the main controller for providing a predetermined gas to the person per predict time interval; and a data output unit electrically connecting with the main controller for receiving the data transmitted from the main controller and outputting the data.

According to the above-mentioned system, the ventilator receives the instruction emitted by the main controller or be inputted therein by manual work to provide a predetermined gas to the person to be measured per predict time interval.

According to the above-mentioned method, the data output unit outputs a curve of the blood oxygen saturation value corresponding to the predict time point, or velocity of the blood circulation of the person to be measured.

### BRIEF DESCRIPTION OF THE DRAWINGS

These and other features and advantages of the various embodiments disclosed herein will be better understood with respect to the following description and drawings, in which like numbers refer to like parts throughout, and in which:

Figure 1 is a block diagram of a method of measuring blood circulation velocity according to an exemplary embodiment of the present invention;

Figures 2A and 2B are curve diagrams of gases provided by a ventilator corresponding to time points according to the method of figure 1;

Figure 3 is a curve diagram of blood oxygen saturation values recorded according to a plurality of time points by using the method of figure 1;

Figure 4A is a block diagram of a system of measuring blood circulation velocity according to an exemplary embodiment of the present invention; and

Figure 4B is a block diagram of another system of measuring blood circulation velocity according to an exemplary embodiment of the present invention.

### DETAILED DESCRIPTION

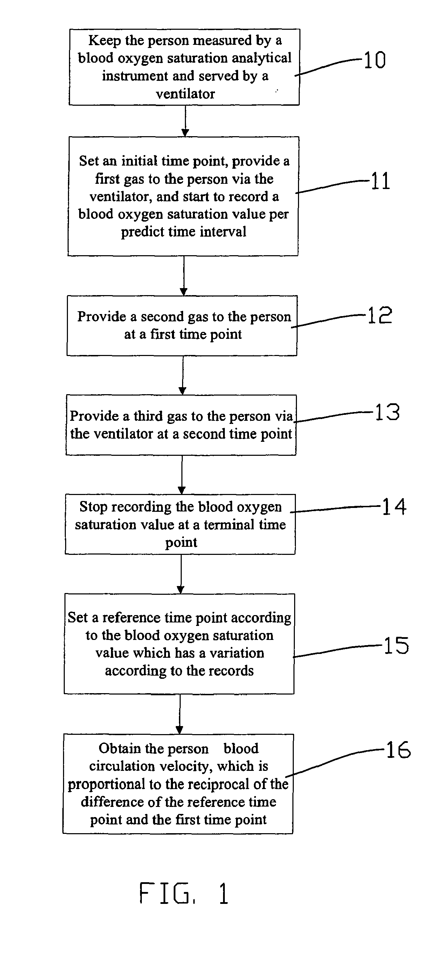

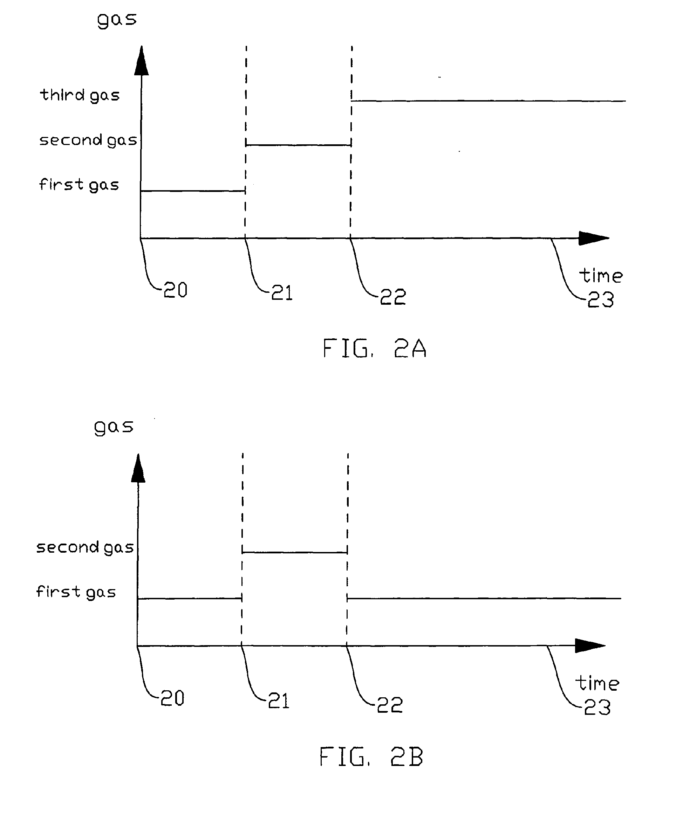

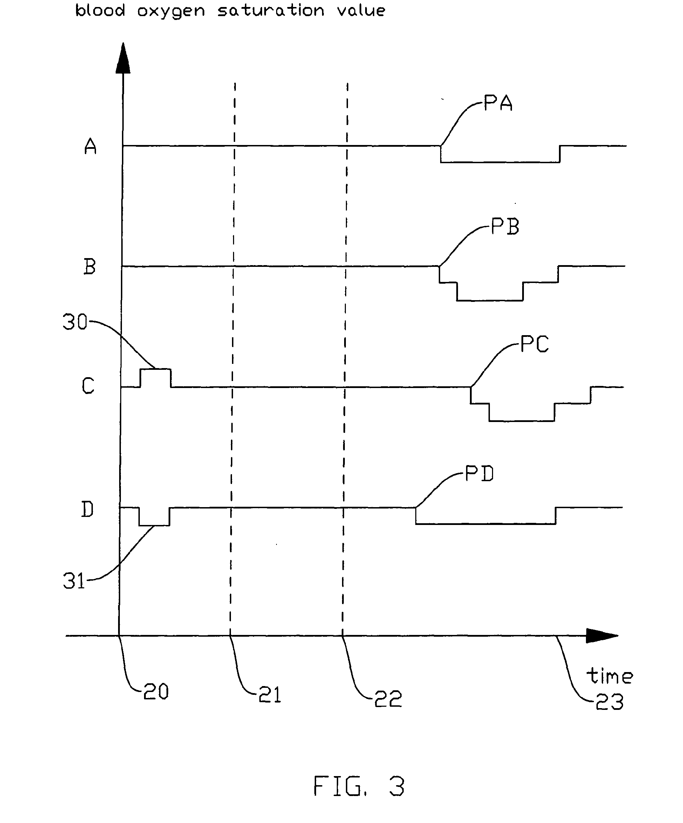

Referring to figures 1, 2A, 2B, and 3, a block diagram of a method of measuring blood circulation velocity according to an exemplary embodiment of the present invention; figures 2A and 2B are curve diagrams of gases provided by a ventilator corresponding to time points according to the method of figure 1; and figure 3 shows four sets of curve diagrams of blood oxygen saturation values recorded according to a plurality of time points by using the method of figure 1. The detail steps of the method are explained as follows.

Firstly, according to step 10 of figure 1, keep the person measured by a blood oxygen saturation analytical instrument and served by a ventilator. The blood oxygen saturation analytical instrument is used to detect the blood oxygen saturation value of the person to be measured, which may be not intrusive type blood oxygen saturation instrument, such as OXY100C. This kind of the instrument would not make the person to induce destructive injury, and it only needs to tightly clip the finger tip of the person to be measured for measuring the blood oxygen saturation values thereof. The method should not be limited with this limitation, and the intrusive type blood oxygen saturation instrument can also be used. The ventilator is use for providing predetermined gas to the person to be measured.

After that, set an initial time point 20, provide a first gas to the person via the ventilator, and start to record a blood oxygen saturation value per predict time interval according to step 11 of figure 1. The first gas can be selected from the group of oxygen, air, and the combination of oxygen and air. Then the curve of the blood oxygen saturation values is approximate to a horizontal straight line corresponding to the x-coordinate of time, referring to curves A and B of figure 3.

Then, provide a second gas to the person at a first time point 21, such as at about 20th second, according to step 12 of figure 1. Wherein, the second gas is different to the first gas, which can be selected form the group of carbon monoxide (CO), nitrogen (N2), carbon dioxide (CO2), the gas without oxygen, and the combination thereof. After that, provide a third gas to the person via the ventilator at a second time point 22, such as at about 40th second, according to step 13 of figure 1. Wherein, the third gas is different to the second gas. The curve of the gas provided to the person via the ventilator according to the time points is shown in figure 2A. Preferably, the third gas is the same as the first gas, that is, make the person come back to status of breathing the first gas, the curve of the gas provided to the person via the ventilator according to the time points is shown in figure 2B. Then, stop recording the blood oxygen saturation value at a terminal time point 23, such as at about 90the second, according to step 14 of figure 1. The terminal time point 23 may be different corresponding to different detecting points, such as finger tip, toe tip, or the like. Properly set a detecting point, it can obtain a plurality of blood oxygen saturation value samples to form a curve, such as curve A or curve B of figure 3. The first time point 21 may not be limited to be at about 20th second, the second time point 22 may not be limited to be at about 40th second, and the terminal time point 23 may not be limited to be at about 90th second. However, the second time point is preferred being set in the range of 25th second ~ 30th second when the second gas is carbon monoxide to prevent the person to be measured from poisoned.

After that, set a reference time point according to the blood oxygen saturation value which has a variation according to the records, according to the step 15 of figure 1. Such as the PA point of figure 3, it denotes that the blood oxygen saturation value is decreased at PA point, and then the time point of the PA point is taken as the reference time point. For similar reasons, PB point of figure 3 also denotes that the blood oxygen saturation value is decreased thereat, which may also be taken as the reference time point. Then, it can obtain the person's blood circulation velocity, which is proportional to the reciprocal of the difference of the reference time point and the first time point, according to the step 16 of figure 1.

According to the method above-mentioned, the theory of this method is that when the person to be measured breathes different gas provided by the ventilator at different time points, the blood oxygen quantity of the lung bubble of the person is changed accordingly. Due to the second gas can be the gas without oxygen, then the blood oxygen quantity of the lung bubble of the person is decreased immediately. But the blood in the body of each of the person has a different velocity, the reducing blood oxygen saturation value due to the decreasing of the blood oxygen quantity of the lung bubble would response at the detecting points, such as finger tip or the like after a certain time period, to be obtained by the blood oxygen saturation analytical instrument. The time period of this process is the reference time point minus the first time point, and the reciprocal of this time period is proportional to the blood circulation velocity of the person to be measured. It is hard to exactly measure the flowing distance of the blood between the lung bubble and the finger tip of the person to be measured. However, the flowing distance of a certain person is the same, so the emersion of this method of measuring the blood circulation velocity reference value is very high, which can easily and exactly be applied.

Moreover, though it is hard to exactly measure the flowing distance of the blood between the lung bubble and the finger tip of the person to be measured, the flowing distance of the blood of the person is proportional to the height thereof. Then it may set up a reference distance table, in which, the reference distance is proportional to the height of the person to be measured. So the blood circulation velocity value of the person can be obtained by using the reference distance dividing the time of the difference of the reference time point and the first time point. Such that, the value of the blood circulation velocity measured by the method according to the present invention may be a valid reference value. On the other hand, it can directly set up a curve diagram of the relative variables relative to the blood circulation velocity, and then the person can obtain the blood circulation velocity by comparing the above-mentioned reference value of the blood circulation velocity to the curve diagram. The relative variables may be relative to sex, age, height, body temperature, blood pressure, red corpuscle, RBC distribution width, deformability of erythrocytes, hematocrit, blood viscosity, basal metabolism, blood vessel diameter etc. It also can directly set up a blood velocity normal distribution table of a healthy person, and then the person to be measured can conveniently look up the healthy index of the blood circulation velocity in the blood velocity normal distribution table.

For improving the veracity of this method, preferably, if the blood oxygen saturation value at the first time point 21 is different to that at the initial time point 20, referring to the variation points 30 and 31 of figure 2, it denotes that the person to be measured doest not act a normal breathing status, the blood circulation velocity measured under this condition is not proper, even if it can obtain the points PC and PD afterwards, it should make the record of the blood oxygen saturation values invalidly. This step can improve the emersion of the method according to the present invention.

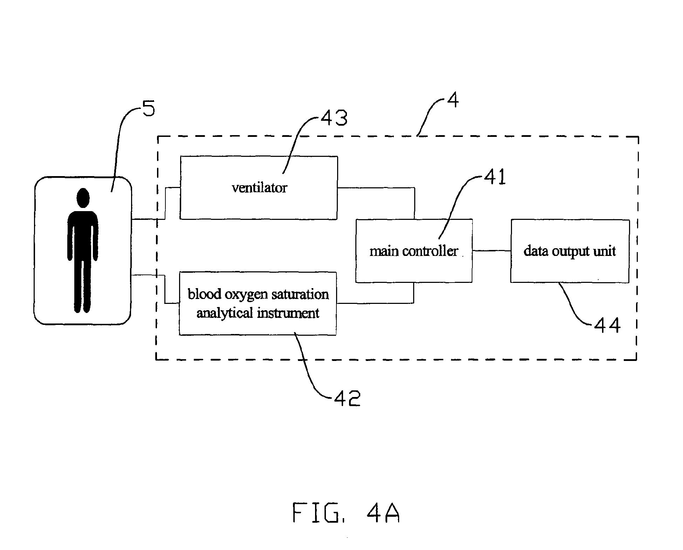

Referring to figure 4A, a block diagram of a system of measuring blood circulation velocity of a person to be measured according to an exemplary embodiment of the present invention is shown. The system 4 includes a main controller 41, a blood oxygen saturation analytical instrument 42 electrically connecting with the main controller 41 for detecting the blood oxygen saturation value of the person 5 to be measured per predict time interval and transmitting the blood oxygen saturation value to the main controller 41, a ventilator 43 electrically connecting with the main controller 41 for providing a predetermined gas to the person 5 per a predict time interval, the curve diagrams of gases provided to the person 5 via the ventilator 43 corresponding to time points are shown in figures 2A and 2B, and a data output unit 44 electrically connecting with the main controller 41 for receiving the data transmitted from the main controller 41 and outputting the data. Preferably, the data output unit 44 is not only capable of directly outputting curve diagrams of blood oxygen saturation values according to a plurality of time points of the person 5 to be measured, but also capable of directly showing the blood circulation velocity that enable the person 5 to rapidly obtain the values.

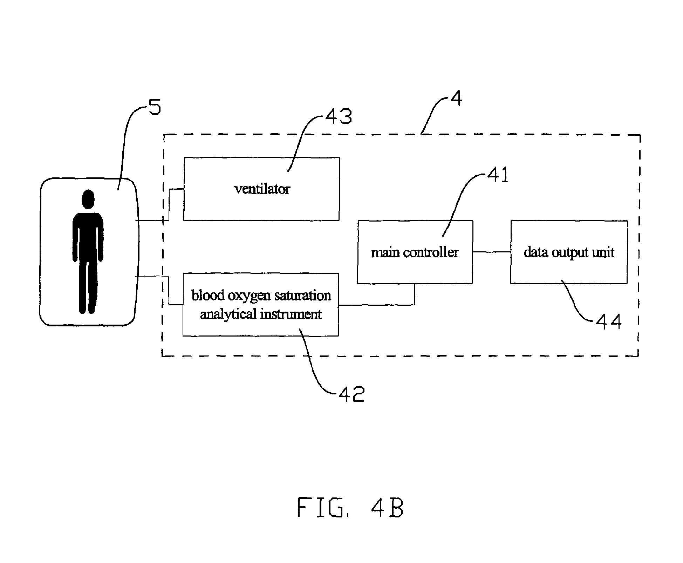

Referring to figure 4B, a block diagram of another system of measuring blood circulation velocity of a person to be measured according to an exemplary embodiment of the present invention is shown. The system 4 has a similar structure to the above-mentioned system. However, the ventilator 43 is operated by manual work or other way different from operating by the main controller 41. The ventilator 43 can provide different gas to the person 5 according to the predict time interval, and function as setting a first and a second time points.

Furthermore, the main controller 41 has the function of auto-recording, which may be realized by hardware, software, or firmware. The main controller 41 is used to record the blood oxygen saturation values according to the time via the blood oxygen saturation analytical instrument for forming the curve diagrams of figure 3. Meanwhile, the main controller 43 has the function of calculating, it can calculate the blood circulation velocity or the reference values of the blood circulation velocity and then output via the data output unit.

The ventilator 43 can receive an instruction emitted by the main controller 41 or be inputted therein by manual work to provide a predetermined gas to the person 5 to be measured per predict time interval.

In addition, the main controller may have the function of memory, for storing the reference distance, the curve diagram of the relative variables relative to the blood circulation velocity, and the blood velocity normal distribution table, which are taken as base sources for comparing.

According to above-mentioned, the method and the system of measuring the blood circulation velocity according to the present invention only use a blood oxygen saturation analytical instrument and a ventilator, which has lower cost and high emersion.

The above description is given by way of example, and not limitation. Given the above disclosure, one skilled in the art could devise variations that are within the scope and spirit of the invention disclosed herein, including configurations ways of the recessed portions and materials and/or designs of the attaching structures. Further, the various features of the embodiments disclosed herein can be used alone, or in varying combinations with each other and are not intended to be limited to the specific combination described herein. Thus, the scope of the claims is not to be limited by the illustrated embodiments.

## Claims

1. A method of measuring blood circulation velocity comprising the steps of:
keeping a person measured by a blood oxygen saturation analytical instrument and served by a ventilator;
setting an initial time point, providing a first gas to the person via the ventilator, and starting to record a blood oxygen saturation value per predict time interval;
providing a second gas to the person at a first time point;
providing a third gas to the person at a second time point;
stopping recording the blood oxygen saturation value at a terminal time point;
setting a reference time point according to the blood oxygen saturation value which has a variation according to the records; and
obtaining the person's blood circulation velocity, which is proportional to the reciprocal of the difference of the reference time point and the first time point.

2. The method as claimed in claim 1, wherein the first gas is different to the second gas.

3. The method as claimed in claim 2, wherein the first gas is selected from the group of oxygen, air, and the combination of oxygen and air.

4. The method as claimed in claim 2, wherein the second gas is selected form the group of carbon monoxide (CO), nitrogen (N2), carbon dioxide (CO2), the gas without oxygen, or the combination thereof.

5. The method as claimed in claim 1, wherein the first gas is the same as the third gas.

6. The method as claimed in claim 1, wherein the blood circulation velocity of the person to be measured is obtained by using a reference distance which is proportional to a height of the person dividing the difference of the reference time point and the first time point.

7. The method as claimed in claim 1, wherein a blood circulation velocity reference value is the reciprocal of the difference of the reference time point minus the first time point, and the blood circulation velocity is obtained by comparing the blood circulation velocity reference to a curve diagram of relative variables relative to blood circulation velocity.

8. The method as claimed in claim 1, wherein the record of the blood oxygen saturation value is invalid if the blood oxygen saturation value at the first time point is different to that at the initial time point.

9. A system of measuring blood circulation velocity, comprising:
a main controller;
a blood oxygen saturation analytical instrument electrically connecting with the main controller for detecting the blood oxygen saturation value of the person to be measured per predict time interval and transmitting the blood oxygen saturation value to the main controller;
a ventilator providing a predetermined gas to the person per predict time interval; and
a data output unit electrically connecting with the main controller for receiving the data transmitted from the main controller and outputting the data.

10. The system as claimed in claim 9, wherein the ventilator receives an instruction and then provides a predetermined gas to the person to be measured per predict time interval.

11. The system as claimed in claim 10, wherein the ventilator is electrically connecting with the main controller, and the instruction is emitted by the main controller.

12. The system as claimed in claim 10, wherein the instruction is inputted into the ventilator by manual work.

13. The system as claimed in claim 9, wherein the data output unit outputs a curve of the blood oxygen saturation value corresponding to the predict time point.
